Europäisches Patentamt

(19) European Patent Office

Office européen des brevets

(11) Publication number: **0 211 767 B1**

# EUROPEAN PATENT SPECIFICATION

(45) Date of publication of patent specification: 06.03.91   (51) Int. Cl.5. **C12N 15/81**, C12N 1/19, //(C12N1/19,C12R1:85)

(21) Application number: 86401739.7

(22) Date of filing: 04.08.86

(54) Method for increasing recombinant protein production in yeast species of the genus saccharomyces.

(30) Priority: 05.08.85 US 762596

(43) Date of publication of application:
25.02.87 Bulletin 87/09

(45) Publication of the grant of the patent:
06.03.91 Bulletin 91/10

(84) Designated Contracting States:
CH DE FR GB IT LI NL

(56) References cited:
EP-A- 0 106 828
EP-A- 0 120 551
WO-A-86/07091

NATURE, vol. 298, no. 5872, July 22, 1982
(London, New York); P. VALENZUELA et
al.:"Synthesis and assembly of hepatitis B
virus surface antigen particles in yeast", pp.
347-350

(73) Proprietor: MERCK & CO. INC.
126, East Lincoln Avenue P.O. Box 2000
Rahway New Jersey 07065-0900(US)

(72) Inventor: **Ellis, Ronald W.**
1407 Edgeville Road
Overbrook Hills Pennsylvania 19151(US)
Inventor: **Montgomery, Donna L.**
9 Hickory Lane
Chalfont Pennsylvania 18914(US)
Inventor: **Schultz, Loren D.**
421 Oak Drive
Harleysville Pennsylvania 19438(US)
Inventor: **Broach, James R.**
84 MacLean Circle
Princeton New Jersey 08540(US)

(74) Representative: **Warcoin, Jacques et al**
Cabinet Régimbeau 26, avenue Kléber
F-75116 Paris(FR)

## Description

### BACKGROUND OF THE INVENTION

Yeast species of the genus Saccharomyces have proven to be very versatile as a host for the expression of a wide variety of recombinant DNA-derived proteins. Owing to their ability to glycosylate foreign proteins and to enable the folding of many proteins to a conformation similar to that of the derived nature protein, yeast, e.g. , S. cerevisiae , have become the host species of choice for the microbial expression of a growing number of proteins with a range of biological functions.

Many industrial laboratories have been utilizing S. cerevisiae as a host for the production of biologicals. An example of such an application is the production of hepatitis B surface antigen (HBsAg) as a vaccine for diseases induced by hepatitis B virus. Typically, workers have utilized haploid strains with auxotrophic markers which enable them to distinguish a production seed strain from a parental or wild-type strain. Since such markers are recessive, haploid strains with these markers are easier to obtain than the corresponding diploid strains. Nevertheless, ploidy per se should not be an obvious factor in the selection of a particular strain as host for the expression of a foreign recombinant-derived protein.

### OBJECTS OF THE INVENTION

It is an object of the present invention to provide host strains of species from the genus Saccharomyces which will be useful for the increased productivity per unit culture volume of recombinant DNA-expressed proteins. Another object is to provide strains of host species from the genus Saccharomyces which will be useful for the increased productivity per unit culture volume of recombinant DNA-expressed HBsAg. These and other objects of the present invention will be apparent from the following description.

### SUMMARY OF THE INVENTION

The productivity of recombinant HBsAg in S. cerevisiae is higher in diploid strains than in haploid strains. Therefore, such strains are more useful as hosts for the expression of recombinant proteins in species from the genus Saccharomyces in terms of increased yield of protein per unit of culture volume.

### DETAILED DESCRIPTION OF THE INVENTION

The present invention is directed to diploid strains of species from the genus Saccharomyces which are useful for the increased productivity of recombinant DNA-derived proteins. The present invention also is directed to the application of such strains to the increased productivity per unit culture volume of recombinant DNA-derived HBsAg.

A diploid strain of S. cerevisiae is constructed by mating two haploid strains of sex phenotypes α and a . The diploid strain and haploid strain each are transformed by a plasmid which is capable of directing the expression of HBsAg in S. cerevisiae . Transformants are cloned and grown in liquid culture to high cell density. The cells are lysed and assayed for the production of HBsAg by radioimmunoassay. The diploid transformant produces HBsAg to an approximately two-fold higher percentage of total cell protein than does the haploid transformant. Moreover, the diploid transformant produces HBsAg to an approximately seven-fold higher total yield per unit culture volume than does the haploid transformant.

The HBsAg protein is but one example of a foreign recombinant DNA-derived protein expressed in S. cerevisiae . Therefore, it will be obvious to those skilled in the art that any foreign recombinant DNA-derived protein can be expressed in a diploid strain for the purpose of increased expression and volumetric productivity. Conceptually, the use of diploid cells addresses the issues of increased vigor and cell size as compared to the use of haploid cells. There are several well-known methods for producing diploid strains from haploid strains. These methods include mating and protoplast fusion. Therefore, it will be obvious to those skilled in the art that any method for producing a doploid strain can be employed for this purpose.

The genus Saccharomyces is composed of a variety of species. Most commonly used is Saccharomyces cerevisiae , or baker's yeast, as a host for the recombinant DNA-mediated expression of a variety of foreign polypeptides. However, the distinctions among other species of the genus Saccharomyces is not always well-defined and the various species can be used interchangeably for many purposes.

Therefore, it will be obvious to those skilled in the art that the concept of using diploid strains, as demonstrated for S. cerevisiae , extends to other species of the genus Saccharomyces , including but not limited to carlsbergensis , rouxii , montanus , kluyveri , and elongisporus.

The following example illustrates the present invention without, however, limiting the same thereto:

EXAMPLE 1

Production of HBsAg in Haploid and Diploid Strains of S. cerevisiae

A diploid strain of S. cerevisiae was constructed by mating two haploid strains (2150-2-3 mating type a and LL20-11$_2$ mating type $\alpha$). The diploid strain and haploid parental strain each were transformed by the plasmid pHBS56-GAP347/33 (disclosed in EP-A-120 551), capable of directing the expression of HBsAg in S. cerevisiae . This plasmid contains the following functional types of sequences:

(1) selection and amplification in Escherichia coli
(2) selection and amplification in S. cerevisiae
(3) transcriptional initiation and termination in S. cerevisiae
(4) the complete HBsAg coding sequence.

Transformants were cloned and grown in liquid culture in selective media. The cells were lysed with glass beads and assayed for the production of HBsAg by radioimmunoassay (AUSRIA®, Abbott); this activity was compared with the amount of total protein as determined by Lowry assay. The diploid transformant produced HBsAg to an approximately two-fold higher percentage of total cell protein than did the haploid transformant. Moreover, the diploid transformant produced HBsAg to an approximately seven-fold higher total yield per unit culture volume than did the haploid transformant.

## HBsAg production in S. cerevisiae

| Strains | | RIA (µg) Protein (mg) | | RIA (µg) Volume (ml) |
|---|---|---|---|---|
| Haploid (2150-2-3) | | 0.85 | | 1.28 |
| | | 0.63 | | 0.92 |
| | | 1.76 | | 1.36 |
| | | 0.79 | | 1.20 |
| | Mean = | 1.01 | Mean = | 1.19 |
| Diploid (2150-2-3) | | | | |
| (LL20-11$_2$) | | 2.40 | | 10.81 |
| | | 1.75 | | 6.69 |
| | | 2.42 | | 8.36 |
| | | 1.98 | | 6.77 |
| | Mean = | 2.14 | Mean = | 8.16 |

Specific activity of diploid strain is 2.14/1.01 = 2.1-fold higher than that of the haploid

Volumetric productivity of diploid strain is 8.16/ 1.19 = 6.9-fold higher than that of the haploid

## Claims

1. A strain of species from the genus Saccharomyces other than S.uvarum transformed by the presence of a vector containing foreign DNA wherein the strain has a ploidy equal to 2.

2. A strain of species from the genus Saccharomyces wherein the foreign DNA encodes at least a part of the hepatitis B virus.

3. A strain of species from the genus Saccharomyces according to claim 2 wherein the foreign DNA encodes substantially all the hepatitis B virus surface antigen.

4. A strain of species from the genus Saccharomyces according to claim 2 wherein the foreign DNA encodes at least part of the pre-S region of the hepatitis B virus.

5. A diploid strain of species from the genus Saccharomyces obtained from two haploid parental strains either or both transformed by a plasmid capable of directing the expression of at least part of the hepatitis B virus.

**Revendications**

1. Souche d'une espèce du genre Saccharomyces différente de S. uvarum transformée par la présence d'un vecteur contenant de l'ADN étranger dans laquelle la souche a une ploïdie égale à 2.

2. Souche d'une espèce du genre Saccharomyces dans laquelle l'ADN étranger code au moins une partie du virus de l'hépatite B.

3. Souche d'une espèce du genre Saccharomyces selon la revendication 2, dans laquelle l'ADN étranger code sensiblement tout l'antigène de surface du virus de l'hépatite B.

4. Souche d'une espèce du genre Saccharomyces selon la revendication 2, dans laquelle l'ADN étranger code au moins une partie de la région pré-S du virus de l'hépatite B.

5. Souche diploïde d'une espèce du genre Saccharomyces obtenue à partir de deux souches parentales haploïdes, l'une ou les deux étant transformée(s) par un plasmide capable de diriger l'expression d'au moins une partie du virus de l'hépatite B.

**Ansprüche**

1. Stamm einer Art aus der Gattung Saccharomyces, die von S. uvarum verschieden ist, der durch die Gegenwart eines fremde DNS enthaltenden Vektors transformiert ist, worin der Stamm eine Ploidie von gleich 2 hat.

2. Stamm einer Art der Gattung Saccharomyces, worin die fremde DNS für wenigstens einen Teil des Hepatitis B-Virus codiert.

3. Stamm einer Art der Gattung Saccharomyces nach Anspruch 2, worin die fremde DNA für im wesentlichen das ganze Oberflächenantigen des Hepatitis B-Virus codiert.

4. Stamm einer Art der Gattung Saccharomyces nach Anspruch 2, worin die fremde DNS für wenigstens einen Teil der Pre-S-Region des Hepatitis B-Virus codiert.

5. Diploider Stamm einer Art der Gattung Saccharomyces, der aus zwei haploiden Elternstämmen erhalten wurde, von denen einer oder beide durch ein Plasmid transformiert wurden, das die Expression wenigstens eines Teils des Hepatitis B-Virus steuern kann.